# EUROPÄISCHE PATENTANMELDUNG

(11) **EP 2 292 623 A1**
(43) Veröffentlichungstag der Anmeldung: **09.03.2011**
(21) Anmeldenummer: 10171747.8
(22) Anmeldetag: 03.08.2010
(51) Int. Cl.: C07D 487/22, A61P 43/00, A61K 31/555, A61Q 1/02, C09B 67/00, H01G 9/20, H01L 31/101, A61K 31/409

(54) **Acylierte Phthalocyanine**

(30) Priorität: 26.08.2009 DE 102009043862
(71) Anmelder: Karlsruher Institut für Technologie, 76131 Karlsruhe (DE)
(72) Erfinder: Balaban, Teodor Silviu, 76187 Karlsruhe (DE); Balaban, Mihaela Carmen, 76187 Karlsruhe (DE)
(74) Vertreter: Fitzner, Uwe

(57) **Zusammenfassung**

Phthalocyanine der allgemeinen Formel 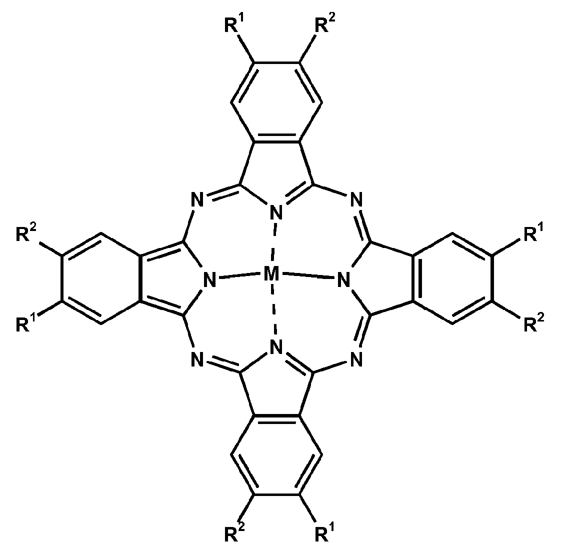 worin R¹ und R² jeweils ausgewählt sind aus der Gruppe bestehend aus Wasserstoff, Alkyl, Acylresten der Formeln -COR und -CRHOH,
mit der Maßgabe, dass mindestens einer und maximal vier der acht Reste aus der Gruppe bestehend aus -COR und -CRHOH ausgewählt werden und mit der Maßgabe, dass falls an einem Benzolring R¹ oder R² ausgewählt sind aus -COR oder -CRHOH, der andere Rest R¹ oder R² an diesem Benzolring für Wasserstoff steht, wobei
R ist gleich CH₃(CH₂)ₙ- mit n = 0 - 15,
M wird ausgewählt aus der Gruppe bestehend aus Mg, Zn, Cd, Cu, Ni, Co, Fe, Pt, Pd, Sn und Mischungen davon,
Alkyl ist gleich CₙH2ₙ₊₁, wobei n = 7 - 14 ist,
Alkoxyl ist gleich OCₙH₂ₙ₊₁, wobei n = 6 - 15,
Verfahren zu deren Herstellung und deren Verwendung.

## Beschreibung

Alle in der vorliegenden Anmeldung zitierten Dokumente sind durch Verweis vollumfänglich in die vorliegende Offenbarung einbezogen (= incorporated by reference in their entirety).

Die vorliegende Erfindung betrifft spezielle Phthalocyanine, Verfahren zu deren Herstellung sowie deren Verwendungen.

### Stand der Technik:

Die natürliche Photosynthese beherrscht Photoneneinfang unter sehr schwachen Beleuchtungen, wie zum Beispiel bei 100 m unter dem Meeresspiegel. Die natürlichen Chromophore, die dafür eingesetzt werden, lassen sich im Labor nicht nachmachen, sind sehr zerbrechlich sowie schwer zu handhaben.

Das Problem der effizienten Lichtsammlung funktioniert in der Natur selbst seit mindestens 2,5 Milliarden Jahren. Bisher wurde lediglich durch das Bauen großer Lichtsammlungsantennen durch Selbstorganisation von völlig synthetischen Molekülen ähnliches nachvollzogen, z.B. in der DE 101 46 970 A1.

Phthalocyanine sind sehr beständige Farbstoffe, die gute Pigmenteigenschaften aufweisen, wie z.B. hohe Absorptionskoeffizienten im sichtbaren Bereich. Bisherige Versuche Phthalocyanine in Farbstoffsensibilisierten Solarzellen einzubauen zeigten nur sehr geringe Effizienz, also geringe Kraftkonversion vom Licht in Strom. Das Problem liegt in der hohen Tendenz zur Aggregation der Farbstoffe, was zu einer Löschung der Fluoreszenz führt. Deshalb bewirken Phthalocyanine üblicherweise nur einen strahlungslosen Desaktivierungsprozess.

Klassische Solarzellen auf Siliziumbasis, sind, obwohl sie eine gute Effizienz wegen der aufwendigen Reinigungsverfahren zeigen, zu teuer um großflächige Anlagen kostengünstig zu bauen. Farbstoffsensibilisierte Solarzellen dagegen zeigen eine deutlich geringere Effizienz, sind aber mit billigen Produktionskosten herstellbar.

### Aufgabe:

Aufgabe der vorliegenden Erfindung ist es, das Problem der effizienten Lichtsammlung, welches in der Natur selbst seit mindestens 2,5 Milliarden Jahren funktioniert, zu lösen. Dabei soll die effiziente Lichtsammlung durch artifizielle Moleküle erfolgen.

Ausgehend vom Vorbild der Bacteriochlorophyllen und durch Erfahrungen mit selbstorganisierenden Porphyrinen sollten entsprechende Moleküle, insbesondere Phthalocyanine gefunden werden, die Erkennungsgruppen aufweisen, welche die Selbstorganisation ermöglichen, sowie die dringend notwendige löslichkeitsfördende Gruppen aufweisen, ohne dass die Komplexität ansteigt, so dass die Herstellungskosten möglichst gering bleiben. Ferner sollte ein Verfahren gefunden werden, dass die einfache Herstellung entsprechender Moleküle ermöglicht.

Es sollte ein Weg gefunden werden, umweltfreundliche Solarenergiegewinnung und Umwandlung der Lichtenergie bereitzustellen.

### Lösung:

Diese Aufgabe wird gelöst durch neue acylierte Phthalocyanine, die gleichzeitig mit löslichkeitsfördernden Gruppen und Erkennungsgruppen ausgestattet sind.

Weiterhin wurde zur Lösung der erfindungsgemäßen Aufgabe das erfindungsgemäße Verfahren zur Herstellung spezieller Phthalocyanine gefunden.

Nicht zuletzt wurden entsprechende Verwendungen für die erfindungsgemäßen Phthalocyanine gefunden.

### Begriffsdefinitionen:

Im Rahmen der vorliegenden Erfindung sind alle Mengenangaben, sofern nicht anders angegeben, als Gewichtsangaben zu verstehen.

Im Rahmen der vorliegenden Erfindung bedeutet der Begriff "Raumtemperatur" eine Temperatur von 20°C. Temperaturangaben sind, soweit nicht anders angegeben, in Grad Celsius (°C).

Sofern nichts anderes angegeben wird, werden die angeführten Reaktionen bzw. Verfahrensschritte bei Normaldruck/Atmosphärendruck, d.h. bei 1013 mbar, durchgeführt.

Im Rahmen der vorliegenden Erfindung wird unter "Löslichkeit" die Menge eines Stoffes verstanden, die sich in einer bestimmten Menge eines bestimmten Lösungsmittels, bei einer bestimmten Temperatur gerade noch lösen lässt. In der vorliegenden Erfindung wird die Löslichkeit dabei in g/l, für ein bestimmtes Lösungsmittel und für eine Temperatur von 20°C angegeben. Als gut löslich werden im Rahmen der vorliegenden Erfindung Stoffe mit einer Löslichkeit von 3 bis 10 g/l, als schlecht löslich Stoffe mit einer Löslichkeit von kleiner als 0,1 g/l verstanden.

### Detaillierte Beschreibung:

Im Rahmen der vorliegenden Erfindung wurden Phthalocyanine der allgemeinen Formel 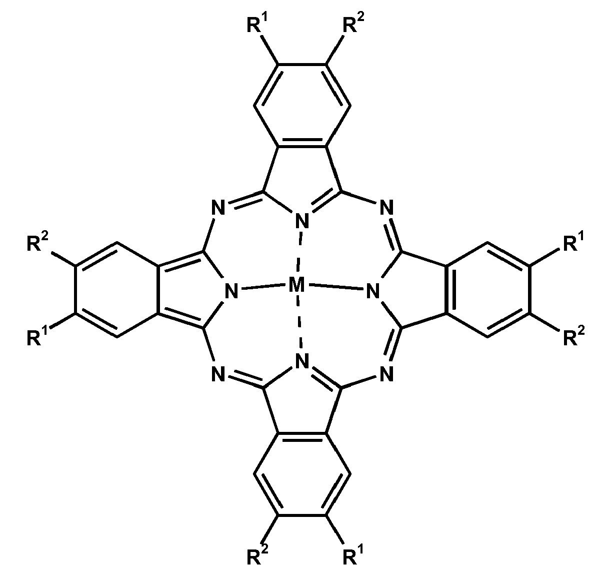 gefunden, worin R¹ und R² jeweils ausgewählt sind aus der Gruppe bestehend aus Wasserstoff, Alkyl, Alkoxyl, Acylresten der Formeln -COR und -CRHOH,
- mit der Maßgabe, dass mindestens einer und maximal vier der acht Reste aus der Gruppe bestehend aus -COR und -CRHOH ausgewählt werden und
- mit der Maßgabe, dass falls an einem Benzolring R¹ oder R² ausgewählt sind aus -COR oder -CRHOH, der andere Rest R¹ oder R² an diesem Benzolring für Wasserstoff steht, wobei
- R ist gleich CH₃(CH₂)ₙ- mit n = 0 - 15, bevorzugt n = 5 - 15,
- M wird ausgewählt aus der Gruppe bestehend aus Mg, Zn, Cd, Cu, Ni, Co, Fe, Pt, Pd, Sn und Mischungen davon, bevorzugt ausgewählt aus der Gruppe bestehend aus Mg, Zn, Cd, Cu, Ni und Mischungen davon
- Alkyl ist gleich CₙH₂ₙ₋₁, wobei n = 7 - 14 ist,
- Alkoxyl ist gleich OCₙH₂ₙ₊₁, wobei n = 6 - 15 ist.

In einer Variante der vorliegenden Erfindung ist das Phthalocyanin diacyliert und n ist jeweils zwischen 5 und 15.

In einer Variante der vorliegenden Erfindung werden CH₃(CH₂)₅- und/oder CH₃(CH₂)₁₁- als Reste R bevorzugt.

In einer Variante der vorliegenden Erfindung ist n = 7 - 15.

Alle Versuche, unsubstituierte Phthalocyanine durch die wohl bekannte Friedel-Crafts-Acylierung zu derivatisieren blieben erfolglos. Demgemäß wurde im Rahmen der vorliegenden Erfindung ein neues Verfahren zur Herstellung von acylierten Phthalocyaninen gefunden.

Um eine hoch geordnete supramolekulare Anordnung der Chromophore zu gewährleisten, dürfen die Erkennungsgruppen nur in bestimmten Positionen vorkommen.

Um dieses Problem zu lösen wurde im Rahmen der vorliegenden Erfindung ein längeren Syntheseweg optimiert, der über mehrere Stufen abläuft.

Eine Synthesemöglichkeit, um die Erkennungsgruppen nur in bestimmten Positionen zu haben ist das erste erfindungsgemäße Herstellverfahren gemäß folgendem Schema:

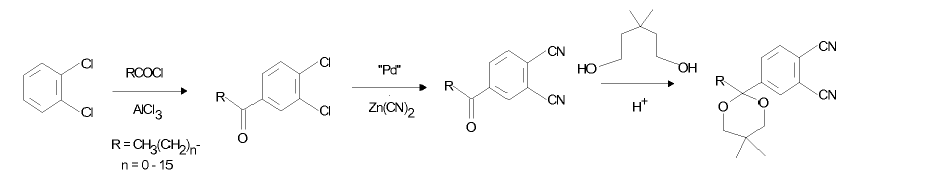

Dieses Syntheseprinzip ist neu, wobei die letzte Stufe, d.h. die Einführung einer Schutzgruppe, optional ist und nur angewendet wird, wenn im Folgeprozess starke Basen (wie Alkalimetallalkoxylate) verwendet werden (für DBU ist es z.B. nicht notwendig).

Der Hauptgrund weshalb keine Acylphthalocyanine in der Literatur beschrieben worden sind, besteht darin, dass die Kondensationsbedingungen, die zum Aufbau des Makrozyklus dienen, prinzipiell inkompatibel mit einer reaktiven Carbonylgruppe sind. Im Rahmen der vorliegenden Erfindung wurde in überraschender Weise gefunden, wie die Carbonylgruppe geschützt werden kann, und wobei die Schutzgruppe gleichzeitig in guten Ausbeuten angebracht und nach der gewünschten Kondensation zum Phthalocyanin, wieder entfernt werden konnte.

Ein erstes Merkmal des ersten erfindungsgemäßen Verfahrens ist die Friedel-Crafts-Acylierungen von *ortho*-Dichlorbenzol mit Acylresten R = CₙH₂ₙ₊₁ mit n > 6. Es ist erstaunlich, dass solche 4-Acyl-1,2-dichlorbenzole nicht vorher beschrieben worden sind. Dies ist wahrscheinlich zurückzuführen auf die geringe Reaktivität, die im Rahmen der vorliegenden Erfindung aber durch hohe Temperaturen, insbesondere zwischen 100°C und 120°C, längere Reaktionszeiten von 5 Stunden und mehr und sehr aktives Aluminiumchlorid (sehr aktives AlCl₃ bedeutet hier neues bzw. wasserfreies AlCl₃) als Friedel-Crafts-Acylierungs- Reagenz/Katalysator überwinden konnten

Ein zweites Merkmal des Syntheseweges des ersten erfindungsgemäßen Verfahrens ist eine neue palladiumkatalysierte Dicyanierung von 4-Acyl-1,2-Dichlorbenzolderivaten. Obwohl Synthesen von Nitrilen von aromatischen Halogenverbindungen bekannt sind, wurden bisher noch keine Phthalodinitrile als solche hergestellt. Phthalodinitrile dienen als Grundbaustein in der Phthalocyaninsynthese der vorliegenden Erfindung. Die Palladiumkatalyse ist kompatibel mit Acylgruppen, so dass auch in diesem Fall keine Schutzgruppen notwendig sind. Für diese Reaktion wird der Pd-Katalysator in situ hergestellt aus dem Pd-Komplex (Tris-(dibenzylidenaceton)-dipalladium(0)) und Ferrocen (1,1'-Bis-(diphenylphosphino)-ferrocen), falls gewünscht kann in einer Variante aktiv Zink (vor der Reaktion mit HCI behandelt) zugegeben werden. Die Reaktion wird während 2 bis 8 Stunden, bevorzugt 3 bis 5 Stunden, insbesondere bevorzugt 3,5 bis 4,5 Stunden bei Temperaturen von 80 bis 160, bevorzugt 100 bis 140, insbesondere bevorzugt 130 bis 150°C durchgeführt. Ein drittes Merkmal des ersten erfindungsgemäßen Verfahrens ist es, solche Reaktionsbedingungen gefunden zu haben, die eine Acylgruppe nicht angreifen, so dass die zusätzlichen Stufen, die mit den Schutzgruppen verbunden sind, vermieden werden können.

Die 4-Acyl-1,2-dichlorbenzole, die 4-Acylphthalodinitrile und deren Acetale wobei der Acylrest jeweils eine Alkylkette CH₃(CH₂)ₙ mit n = 7 - 15 aufweist, sind Zwischenprodukte im erfindungsgemäßen Verfahren, zudem aber auch an sich neue Verbindungen. Sie eignen sich sehr gut für die Phthalocyaninsynthesen, insbesondere in dem oben aufgezeigten erfindungsgemäßen Verfahren.

Bezogen auf die Tatsache, dass die 4-Substitution gegeben ist, sind bei der Kondensation der 4-Acylphthalodinitrile, insgesamt nur die vier Isomere der nachfolgenden Darstellung zu erwarten.

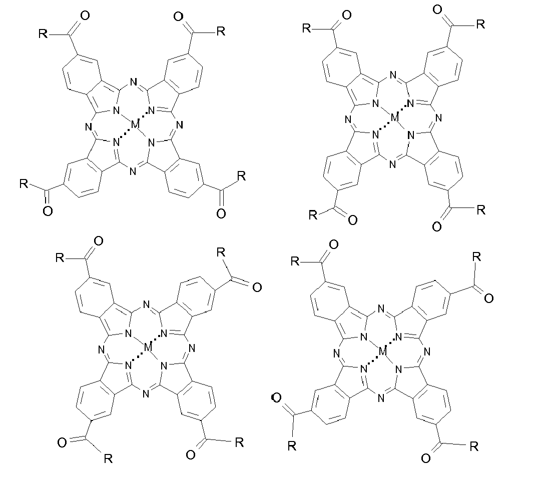

Bei einer gemischten Kondensation mit einem zweiten Pthalodinitril, das sowohl mono- als auch disubstituiert sein kann, gemäß folgender Darstellung, 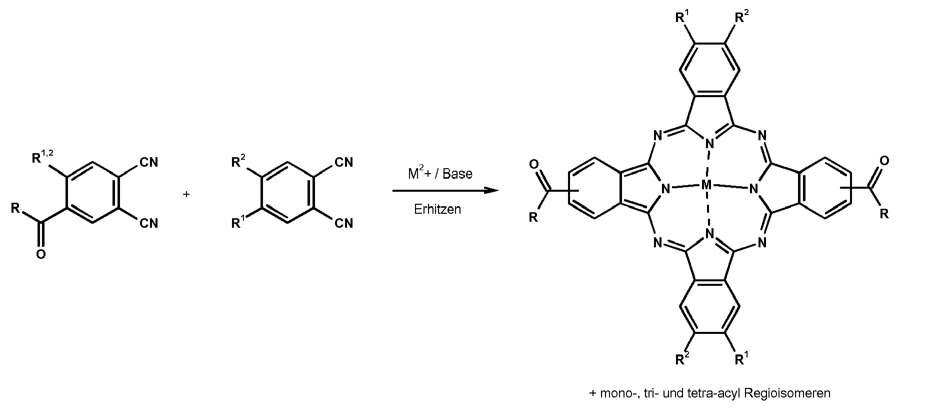 R^{1,2} = Wasserstoff,
R¹, R², Alkyl und M wie oben beschrieben
erhält man ein Gemisch aus nicht-, mono-, di-, tri- und die vier tetra-acylierten Isomeren der obigen Darstellung.

Als Base können dabei im Rahmen der vorliegenden Erfindung verschiedenste gebräuchliche Basen eingesetzt werden, inklusive Alkalimetallalkoxylaten, bevorzugt Li-Pentanolat, Na-Ethanolat, Na-Isopropanolat, In einer Variante werden nicht-nukleophile Basen ausgewählt aus der Gruppe bestehend aus 1,8-Diazabicyclo[5.4.0]undec-7-en (DBU), Pyridin, Quinolin, 2,6-Di-tert-butylpyridin und Mischungen davon verwendet. In einer Variante der vorliegenden Erfindung wird bevorzugt DBU als Base eingesetzt.

Es kommen keine Acylgruppen in der alpha-Position vor, wodurch die Isomerenmischung insgesamt weniger komplex ist und mittels HPLC eine Trennung und Gewinnung der gewünschten Phthalocyanine, bevorzugt Diacylphthalocyanine, erfolgen kann.

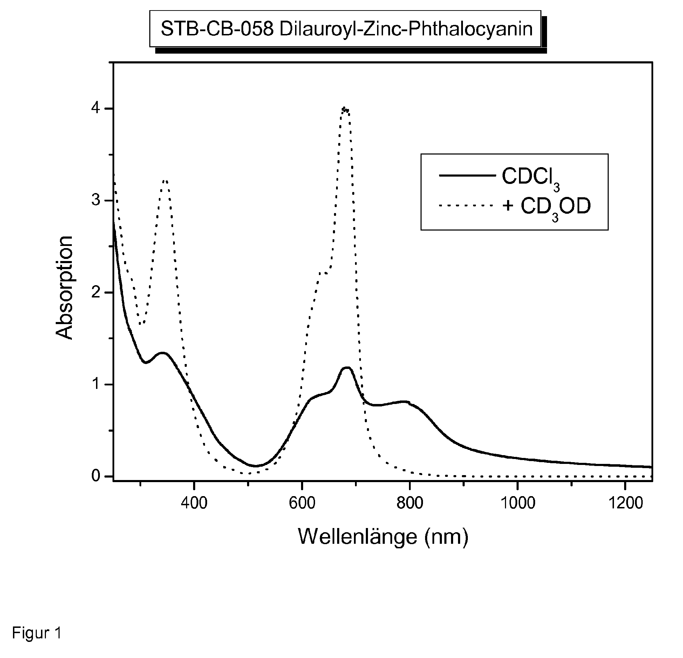

Bereits die Diacylphthalocyanine zeigen eine sehr hohe Tendenz zur Selbstorganisation. Dieses ist durch breite und rotverschobene Absorptionspektren zu erkennen (Figur 1).

Im Rahmen der vorliegenden Erfindung wurde überraschend gefunden, dass die Selbstanordnung schon in Lösungsmitteln von mittleren Polarität inklusive Dichlormethan oder Chloroform eintritt.

Dies ist bei aus dem Stand der Technik bekannten Porphyrinanaloga nicht der Fall, die bestenfalls in nichtpolaren Lösungsmitteln wie n-Hexan, Cyclohexan oder n-Heptan Selbstanordnung zeigen.

Es ist dabei ein besonderes Merkmal der vorliegenden Erfindung, dass es gelungen ist Erkennungsgruppe (entspricht R in den Formeln) und löslichkeitsfördernde Gruppe (entspricht der -CO- bzw. -COH- Gruppe) in einem Rest zu vereinen, wodurch deren Einführung auf elegante und einfache Weise möglich wurde.

Durch Zugabe geringer Mengen von das zentrale Metallatom koordinierenden Lösungsmitteln, inklusive Alkoholen wie bevorzugt Methanol oder Ethanol, oder Pyridin, können die Assemblagen wieder zu Monomeren zersetzt werden.

Dies spiegelt sich in den Absorptionsspektren mit schmalen, intensiven Banden im sichtbaren Bereich (~680 nm) wieder (siehe Figur 1).

Diese Eigenschaft kann genutzt werden, um bestehende Assemblagen an einen anderen Zweck anzupassen, oder fehlerhafte Assemblagen auszugleichen, indem sie zuerst durch Zugabe von Lösemittel zersetzt werden und dann durch gezielte Entfernung oder Ersatz des Lösemittels wieder der Selbstorganisation unterworfen werden.

Als zentrales Metallatom für Solarzellen können im Rahmen der vorliegenden Erfindung insbesondere Zink, oder Magnesium eingesetzt werden, da die guten Fluoreszenzeigenschaften mit hohen Quantenausbeuten beibehalten bleiben. Andere zweiwertige Metalle können auch benutzt werden, allerdings kann es durch den Schweratomeffekt zur Fluoreszenzlöschung kommen, so dass diese in der Regel weniger bevorzugt sind. Es sind aber Anwendungen möglich, wo diese anderen zweiwertigen Metalle vorteilhaft sind.

Weitere erfindungsgemäße Verbindungen die eine hohe Selbstorganisationstendenz aufweisen sind die monoreduzierten Verbindungen der allgemeinen Formel 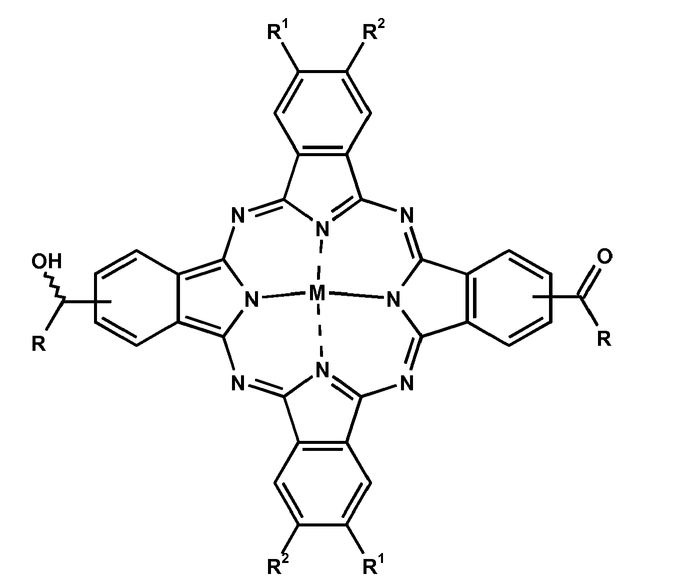 wobei die Reste -COR oder -CRHOH jeweils an 3 oder 4 Stellung des Ringes stehen können, die sich ausgehend von den erfindungsgemäßen Diacylphythalocyaninen sehr einfach und in hohen Ausbeuten herstellen lassen und wobei die Variablen die oben genannten Bedeutungen haben.

In einer Variante der vorliegenden Erfindung wird diese Monoreduktion mit Natriumborhydrid in Methanol/Dichlormethan durchgeführt, und führt dann zu einem Enantiomerengemisch.

In einer Variante der vorliegenden Erfindung kann diese Monoreduktion mit chiralen Borhydriden stereoselektiv durchgeführt werden, wie dies in der Diplomarbeit von Dennis Mössinger "Stereoselektive Monoreduktionen an diacetylsubstituierten Porphyrinen" in Kapitel 3, Seiten 32 bis 47 beschrieben ist.

Die erfindungsgemäßen acylierten Phthalocyanine zeigen zum ersten Mal, dass solch breiten Absorptionsspektren im Wellenlängenbereich von 600 - 950 nm durch die Selbstorganisation zu Stande kommen. Dies war aus dem bisherigen Stand der Technik nicht bekannt.

Die erfindungsgemäßen acylierten Phthalocyanine, die die *künstliche* Lichtsammlung ermöglichen, bieten mit Blick auf die Zukunft umweltfreundliche und großflächige Wirkungsfähigkeit.

Die erfindungsgemäßen acylierten Phthalocyanine, sind löslich in polaren Lösemitteln, insbesondere in leicht polaren, halogenierten Kohlenwasserstoffen inklusive Dichlormethan und Chloroform, insbesondere zu 3 - 10 g/l, und können als Farbstoffe verwendet werden, insbesondere dort wo die klassischen Phthalocyanine versagen, wie z.B. bei der chromatographischen Auftrennung bzw. Reinigung, bei der Mischung in Polymere und Harze. Sehr bekannt sind Phthalocyanine als Pigmente der Autolacke oder in Farbphotokopierern, die durch ihre Brillanz und Beständigkeit andere Pigmente verdrängt haben. Auch für diese Verwendung eignen sich die erfindungsgemäß Phthalocyanine hervorragend.

Die erfindungsgemäßen Phthalocyanine haben die besondere Eigenschaft, sich selbst zu gut geordneten, großen Nanostrukturen zu assemblieren. Dabei gehen die Fluoreszenzeigenschaften nicht verloren. Besonders vorteilhaft ist auch die erhöhte Löslichkeit von 3 - 10 g/l der erfindungsgemäßen Phthalocyanine im Vergleich zu unsubstituierten Phthalocyaninen, die in jedem Lösemittel unlöslich sind.

Die erhöhte Löslichkeit der erfindungsgemäßen Phthalocyanine ermöglicht insbesondere auch ein leichtes nasschemisches Herstellverfahren für selbstorganisierende Assemblagen. Insbesondere geht man dabei so vor, dass man die in polarem Lösemittel gelösten erfindungsgemäßen Phthalocyanine in eine größere Menge eines unpolaren und wasserfreien (da sich Wasser an das zentrale Metallion koordinieren und somit die Assemblagen stören kann) Lösemittels, wie z.B. Kohlenwasserstoffe, wobei dann spontan gut geordnete Assemblagen entstehen.

Der Hauptvorteil der erfindungsgemäßen Phthalocyaninassemblagen ist ein viel breiteres Absorptionsvermögen im Vergleich mit den Monomeren. Eine breite Bande erstreckt sich bis zu 950 nm in den Nachinfrarot Bereich, was für Solarzellen ein besonderes Fenster für Sonnenlichteinfang darstellt. Weiterer Vorteil der erfindungsgemäßen Assemblagen ist die erhöhte Quantenausbeute.

Die erfindungsgemäßen Phthalocyanine und deren Assemblagen können demgemäß hervorragend zur Herstellung von farbstoffsensibilisierten Solarzellen verwendet werden, die gegenüber dem Stand der Technik eine erhöhte Effizienz aufweisen.

Bevorzugte Verwendungen der erfindungsgemäßen Phthalocyanine sind im Rahmen der vorliegenden Erfindung großflächige, hybride Solarzellen, die auch unter schwachen Lichtbedingungen (wie bspw. in Deutschland) effizient arbeiten können.

Weitere bevorzugte Verwendungen der erfindungsgemäßen Phthalocyanine sind in der Pigmentindustrie inklusive Kosmetikindustrie (grüne Nuancen sind sehr begehrt besonders bei amerikanischen dunkelhäutigen Frauen) oder in der Medizin für die Photodynamische Therapie (PDT).

In einer Variante der vorliegenden Erfindung werden die erfindungsgemäßen Phthalocyanine in lichtempfindlichen Schichten, bevorzugt von Solarzellen, als aktive Lichtsammlungsschicht, insbesondere mit per Selbstorganisation angeordneten erfindungsgemäßen Phthalocyaninen, verwendet.

Darüber hinaus können die erfindungsgemäßen Phthalocyanine auch in anderen Anwendungen, eingesetzt werden.

Gegenstand der vorliegenden Erfindung sind demgemäß auch Assemblagen enthaltend oder bestehend aus erfindungsgemäßen acylierten Phthalocyaninen.

Gegenstand der vorliegenden Erfindung sind demgemäß auch Solarzellen enthaltend die erfindungsgemäßen acylierten Phthalocyanine.

Die verschiedenen Ausgestaltungen der vorliegenden Erfindung, z.B. diejenigen der verschiedenen abhängigen Ansprüche, können dabei in beliebiger Art und Weise miteinander kombiniert werden.

Die Erfindung wird nun unter Bezugnahme auf die folgenden nichtlimitierenden Beispiele erläutert.

### Beispiele:

### Herstellung von 1,2-Dichlor-4-lauroyl-benzen

In einem trocknen 250 ml Dreihalskolben mit Thermometer, Rückflusskühler (unter Stickstoff) und Tropftrichter wurden 17.73 g AlCl₃ (0.133 mol) in 50 ml 1,2-Dichlorbenzen suspendiert und auf 0°C abgekühlt. Unter kräftigem Rühren wurden 31.6 ml Laurinsäurechlorid (0.133 mol) langsam (1 Stunde) zugetropft. Danach ließ man abkühlen, bis die Temperatur die Raumtemperatur erreicht hatte. Anschließend wurde langsam auf ca. 100 - 105°C erhitzt, wobei eine Veränderung der Farbe zu erkennen war (von ocker bis dunkelrot). Anschließend wurde bei dieser Temperatur 5 Stunden lang gerührt und bei Raumtemperatur über Nacht stehengelassen. Zur Zerlegung des Komplexes wurde diese Mischung vorsichtig unter Rühren auf 200 g Eis (5% HCI) gegossen. Das Gemisch wurde zu einer senffarbigen Paste, die in Ethylacetat extrahiert wurde. Dann wurde die organische Schicht im Scheidetrichter abgetrennt und mit gesättigter Natriumhydrogencarbonatlösung (2 x 50 ml) und Wasser bis pH neutral gewaschen. Nach dem Trocknen über Natriumsulfat wurde das Ethylacetat eingedampft. Das restliche 1,2-Dichlorbenzen wurde im Vakuum abdestilliert. Der Rückstand wurde chromatographiert über eine Säule (Länge = 20 cm, Durchmesser = 5 cm) an Silicagel (Merck 0.040 - 0.063 nm) mit Dichlormethan als Eluent.

Man erhielt eine ölige Substanz mit Siedepunkt (bestimmt über Kugelrohrdestillation) von 275°C bei 0.8 mbar.
Ausbeute: 25.64 g, 87%
¹H NMR (300 MHz, CDCl₃) delta (ppm): 8.00 (d, J=2.1 Hz), 7.77 (d, J=2.1 Hz), 7.74 (d, J=2.1 Hz), 7.53 (d, J=2.4 Hz), 7.5 (d, J=2.4 Hz), 2.89 (m), 2.36 (m), 1.24(m), 0.86 (m). ¹³C-NMR (75 MHz, CDCl₃) delta (ppm): 198.08, 137.33, 136.51, 133.16, 130.61, 130.01, 127.03, 38.56, 31.86, 29.57, 29.44, 29.39, 29.29, 29.20, 24.05, 22.64, 14.08.

### Herstellung von 1,2-Dichlor-4-hexanoyl-benzen

Für die Herstellung dieser Verbindung wurde wie bei dem vorstehenden Beispiel vorgegangen. Statt des Laurinsäurechlorids wurde jedoch 18.6 ml Hexansäurechlorid (0.13 mol) eingesetzt.
Ausbeute: 15.02 g, 68.8%.

### Herstellung von 1,2-Dicyan-4-lauroyl-benzen

1,2-Dichlor-4-lauroyl-benzen (1 g, 3.04 mmol), Tris-(dibenzylidenaceton)-dipalladium (0) (Pd₂(dba)₃) (0.22 g, 0.12 mmol, 8 mol-%), 1,1'-Bis-(diphenylphosphino)-ferrocen (dppf) (0.26 g, 0.24 mmol, 16 mol-%), aktiv Zink (0.09 g, 0.72 mmol, 48 mol-%), und Zinkcyanid (0.57 g, 4.8 mmol, 160 mol-%) wurden unter Stickstoff gewogen und in einem trockenen 50 ml-Kolben als Feststoff hinzugefügt. 15 ml N,N-Dimethylacetamid (DMA), welches vorher 5 Minuten mit Stickstoff gespült wurde, wurde über eine Septumkappe zugespritzt. Die Reaktionsmischung wurde unter Stickstoff für 4 Stunden bei 120°C gerührt. Danach wurde diese Reaktionsmischung über Nacht unter Rühren und bei Raumtemperatur stehengelassen. Danach wurde die Reaktionsmischung in einen Scheidetrichter transferiert, mit 100 ml Ethylacetat verdünnt, mit 50 ml NH₄OH (12.5% wässr. NH₃) und gesättigter Natriumchloridlösung gewaschen bis der pH-Wert neutral war. Das Produkt wurde über Natriumsulfat getrocknet und das Lösungsmittel daraufhin am Rotationsverdampfer eingedampft. Es folgte die Chromatographie über eine Säule (Länge = 30 cm, Durchmesser = 3.5 cm) an Silicagel (Merck 0.040 - 0.063 nm) mit einer Mischung Ethylacetat : Hexan = 1 : 2 (v/v) als Eluent.
Ausbeute: 0.5 g, (53%)
¹H NMR (300 MHz, CDCl₃) delta (ppm): 8.34 (d, J=1.2 Hz), 8.27 (d, J=1.8 Hz), 8.25 (d, J=1.8 Hz), 7.95 (d, J=0.3 Hz), 7.92 (d, J=0.3 Hz), 2.98 (t), 2.37 (t), 1.25 (m), 0.87 (t).

### Herstellung von acyliertem Phthalocyanin

In einem trockenen 250 ml Dreihalskolben mit Thermometer, Rückflusskühler (unter Stickstoff) wurden 0.5 g 1,2-Dicyan-4-lauroyl-benzen (1.6 mmol), 0.2 g Phthalsäuredinitril, 150 ml 1-Pentanol und 1.22 g 1,8-Diazabicyclo[5.4.0]undec-7-en (8 mmol) gemischt. Diese Reaktionsmischung wurde für 10 Minuten mit Stickstoff gespült. Danach wurde bis auf 130°C erhitzt, und 0.36 g Zinkacetat (2 mmol) wurden zugesetzt. Es wurde weiter aufgeheizt bis zum Rückfluss und dann die Temperatur für 48 Stunden gehalten. Die Farbe änderte sich in türkis. Das Pentanol wurde im Vakuum abdestilliert. Der Rückstand wurde in Dichlormethan (150 ml) gelöst und mit gesättigter Ammoniumchloridlösung (2 x 50 ml), gesättigter Natriumchloridlösung (50 ml) und Wasser (50 ml) gewaschen. Das Lösungsmittel wurde eingedampft und es blieb ein türkisfarbiger Rückstand.

### Alternative Herstellung von acyliertem Phthalocyanin:

Es wurde wie in dem vorhergehenden Beispiel vorgegangen, mit den folgenden Abweichungen:
Das Reaktionsgemisch wurde unter starkem Rühren mit einer Leistung von 150 mW bei einer Temperatur von 140°C und einem Druck von 73 mbar mikrowellenbehandelt. Dadurch konnte die Reaktionszeit von 48 Stunden auf 5 Minuten reduziert werden.

## Patentansprüche

1. Acylierte Phthalocyanine der allgemeinen Formel 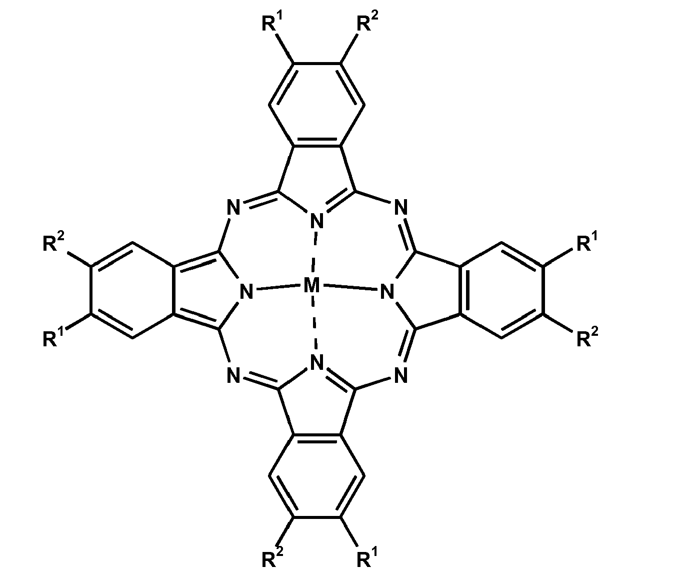 worin R¹ und R² jeweils ausgewählt sind aus der Gruppe bestehend aus Wasserstoff, Alkyl, Alkoxyl, Acylresten der Formeln -COR und -CRHOH, mit der Maßgabe, dass mindestens einer und maximal vier der acht Reste aus der Gruppe bestehend aus -COR und -CRHOH ausgewählt werden und
mit der Maßgabe, dass falls an einem Benzolring R¹ oder R² ausgewählt sind aus -COR oder -CRHOH, der andere Rest R¹ oder R² an diesem Benzolring für Wasserstoff steht, wobei
R ist gleich CH₃(CH₂)ₙ- mit n = 0 - 15,
M wird ausgewählt aus der Gruppe bestehend aus Mg, Zn, Cd, Cu, Ni, Co, Fe, Pt, Pd, Sn und Mischungen davon,
Alkyl ist gleich CₙH₂ₙ₊₁, wobei n = 7 - 14 ist,
Alkoxyl ist gleich OCₙH₂ₙ₊₁, wobei n = 6 - 15 ist.

2. Phthalocyanine nach Anspruch 1, **dadurch gekennzeichnet, dass** sie monoacyliert sind.

3. Phthalocyanine nach Anspruch 1, **dadurch gekennzeichnet, dass** sie polyacyliert (di-, tri- und tetra-acyliert) sind, bevorzugt diacyliert.

4. Phthalocyanine nach Anspruch 1 entsprechend der allgemeinen Formel, 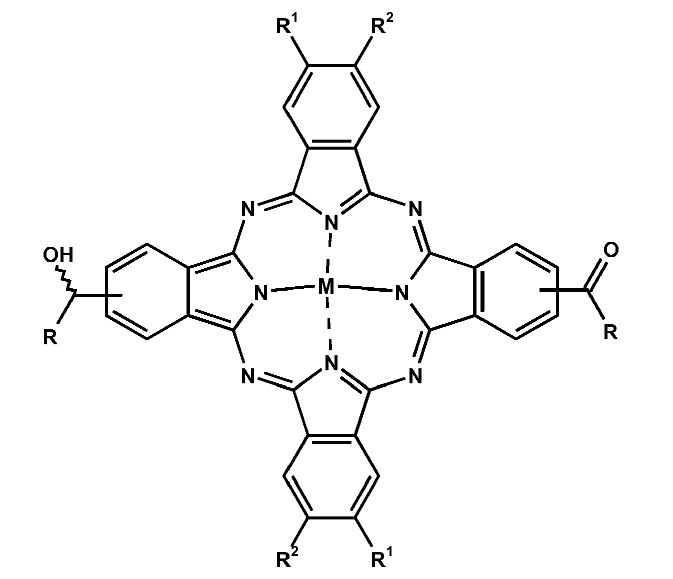 wobei die Reste -COR oder -CRHOH jeweils an 3 oder 4 Stellung des Ringes stehen können.

5. Phthalocyanine nach Anspruch 3, worin R = CH₃(CH₂)ₙ- mit n = 4, 10 oder 14, insbesondere in diacylierter Form.

6. Verfahren zur Herstellung der Phthalocyanine gemäß einem der Ansprüche 1 bis 5, **dadurch gekennzeichnet, dass**
entweder 4-Acylphthalodinitrile miteinander kondensiert werden, oder eine gemischte Kondensation von 4-Acylphthalodinitrilen mit 3,4-disubstituierten Phthalodinitrilen gemäß folgendem Schema 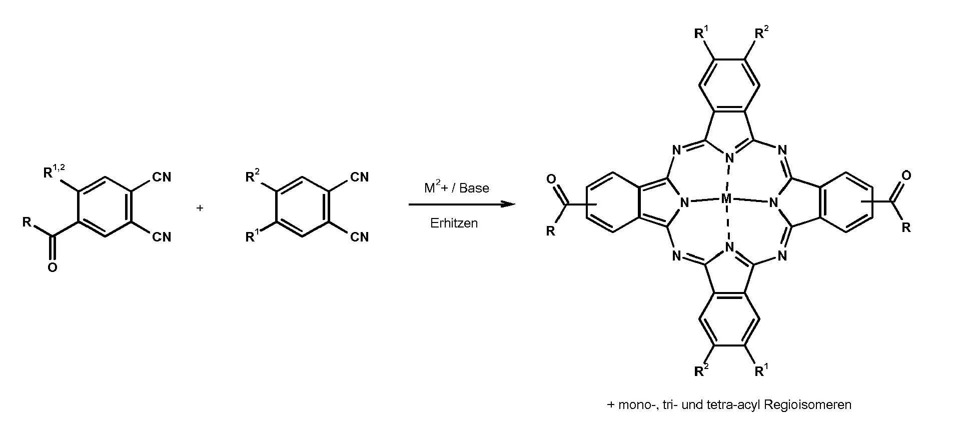 R^{1,2} = Wasserstoff,
R¹, R², Alkyl und M wie oben beschrieben
Base = Alkalimetallalkoxylaten, nicht-nukleophile Basen erfolgt.

7. Verfahren nach Anspruch 6, **dadurch gekennzeichnet, dass** als Phthalodinitrile und/oder deren Acetale solche hergestellt nach folgendem Schema 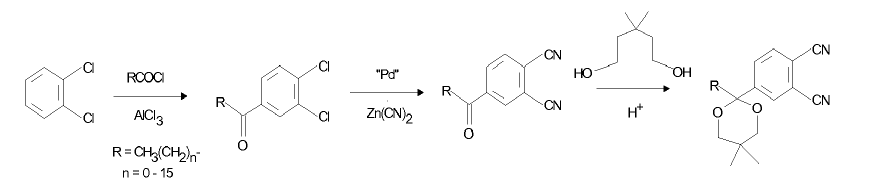 eingesetzt werden.

8. Verfahren zur Herstellung von Phthalodinitrilen und deren Acetalen gemäß folgendem Schema

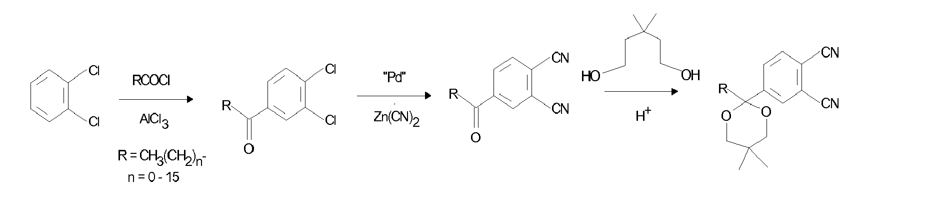

9. Verbindung ausgewählt aus der Gruppe bestehend aus 4-Acyl-1,2-dichlorbenzol, 4-Acylphthalodinitril, 4-(5,5-Dimethyl-1,3-Dioxan-)phthalonitril, wobei der Acylrest jeweils eine Alkylkette CH₃(CH₂)ₙ mit n = 7 - 15 aufweist.

10. Verwendung der Phthalocyanine nach einem der Ansprüche 1 bis 5 in Solarzellen, Lacken, in lichtempfindlichen Schichten als aktive Lichtsammlungsschicht in der Pigmentindustrie inklusive Kosmetikindustrie in der Medizin für die Photodynamische Therapie (PDT), zur Herstellung von farbstoffsensibilisierten Solarzellen.

11. Assemblagen enthaltend oder bestehend aus Phthalocyaninen gemäß einem der Ansprüche 1 bis 5.

12. Solarzellen enthaltend Phthalocyanine gemäß einem der Ansprüche 1 bis 5.
